# EUROPEAN PATENT SPECIFICATION

(11) **EP 2 245 176 B1**
(45) Date of publication and mention of the grant of the patent: **29.08.2012**
(21) Application number: 09704054.7
(22) Date of filing: 23.01.2009
(51) Int. Cl.: C12Q 1/02, C07D 265/38, A01C 1/00, A01H 5/10

(54) **AGENT FOR DETECTING SEED VIABILITY AND A DETECTION METHOD OF SEED VIABILITY USING THE AGENT**
MITTEL ZUM NACHWEIS DER LEBENSFÄHIGKEIT VON SAATGUT UND VERFAHREN ZUM NACHWEIS DER LEBENSFÄHIGKEIT VON SAATGUT UNTER VERWENDUNG DES MITTELS
AGENT DE DÉTECTION DE LA VIABILITÉ DES SEMENCES ET PROCÉDÉ DE DÉTECTION DE LA VIABILITÉ DES SEMENCES UTILISANT LEDIT AGENT

(30) Priority: 23.01.2008 KR 20080006997; 19.12.2008 EP 08172438
(43) Date of publication of application: 03.11.2010
(73) Proprietor: Rhino Research Europe B.V., 7122 JH Aalten (NL); Industrial & Academic Cooperation Foundation, Daegu Uninversity, Gyeongbuk 712-714 (KR)
(72) Inventor: MIN, Tae-Gi, Gyeongsan Gyeongbuk 712-714 (KR)
(74) Representative: Hatzmann, Martin
(86) International application number: PCT/NL2009/050033
(87) International publication number: WO 2009/093905

(56) References cited:
- BYTH HEATHER-ANNE ET AL: "Assessment of a simple, non-toxic Alamar Blue cell survival assay to monitor tomato cell viability" PHYTOCHEMICAL ANALYSIS, vol. 12, no. 5, September 2001 (2001-09), pages 340-346, XP009114719 ISSN: 0958-0344
- JAMNIK POLONA ET AL: "Antioxidative action of royal jelly in the yeast cell" EXPERIMENTAL GERONTOLOGY, vol. 42, no. 7, July 2007 (2007-07), pages 594-600, XP022085158 ISSN: 0531-5565
- DATABASE WPI Week 200216 Thomson Scientific, London, GB; AN 2002-120078 XP002522100 & KR 2001 075 878 A (MIN T G) 11 August 2001 (2001-08-11)
- TIBALLI ROBERT N ET AL: "Torulopsis glabrata: Azole susceptibilities by microdilution colorimetric and macrodilution broth assays" JOURNAL OF CLINICAL MICROBIOLOGY, vol. 33, no. 10, 1995, pages 2612-2615, XP009114760 ISSN: 0095-1137
- KAUFFMAN CAROL A ET AL: "Colorimetric method for susceptibility testing of voriconazole and other triazoles against Candida species" MYCOSES, vol. 42, no. 9-10, November 1999 (1999-11), pages 539-542, XP009114759 ISSN: 0933-7407
- MARRERO PATRICIA ET AL: "Comparison of three chemical tests to assess seed viability: the seed dispersal system of the Macaronesian endemic plant Rubia fruticosa (Rubiaceae) as an example" CHEMOECOLOGY, vol. 17, no. 1, March 2007 (2007-03), pages 47-50, XP009114718 ISSN: 0937-7409

## Description

### FIELD OF THE INVENTION

The present invention is in the field of seed viability testing. More in particular, the invention relates to the detection of metabolites such as carbohydrates and amino acids, discharged from non-viable (non-germinating) seed, in order determine the viability of said seed. The invention further relates to a composition for measuring seed viability and to the use of resazurin as an indicator for seed viability.

### BACKGROUND OF THE INVENTION

The biological quality of a seed is reflected in its capacity to germinate and form a healthy plant. The biological quality of individual seed in a seed lot can vary, and may degenerate during storing. In fact, seed quality deteriorates significantly during prolonged storage and it is not possible to maintain the germination rate of seeds in a seed lot at 100%. It is desirable to provide an indication of the biological quality of seed, that is, by indicating that the seed is a germinating seed, non-germinating seed or an abnormally germinating seed (which results in an abnormal seedling and plantlet). By this indication, the agricultural value (sowing density) or the commercial value of the can be determined.

Seed deterioration is believed to coincide with membrane damage. As a result, a number of compounds, such as soluble sugars, amino acids and electrolytes, leached out from the deteriorated seeds when the seeds are imbibed in water. Seed leaching has been used to assess seed viability by a positive correlation between the amount of leaching from the seed and loss of seed viability. The most common method to detect membrane leakage of seeds is electrical conductivity measurements of seed leachate. As many workers have shown a positive correlation between the conductivity test and seed vigor, the vigor tests using seed leachate have been further developed. A modified method of seed leakage for viability test has been developed by measuring the leakage from individual seeds using spectrophotometric absorbance at 260nm. Also, pH of seed exudate can be detected by adding sodium carbonate and phenolpthalein which changes the soak water to a pink color for viable seeds and no color for non-viable seeds, but seeds have to be cut before imbibition. Recently, a method based on sinapine or amino acid leakage was developed as a single seed nondestructive viability test for *Brassica* species, but the seeds must be pretreated before evaluation. All of these methods do not provide for a method for determining seed viability which can be performed on a single seed (is single seed based) and/or which supports direct detection. In contrast, seeds must be pretreated or cut. Such a method is however sought for. Such a method would be very useful for evaluating seed quality in seed industry, and knowing the state of seed in advance without sowing seed in agriculture works or conduct germination check.

Heretofore examination of seed viability is mainly used through tetrazolium test, this is the method of destroying seed and using the change in color of tetrazolium as indicator, that is, to test whether seed is alive or dead by detecting enzyme activity and hence viability of the seed cell. This method has the problem that the seed is destroyed, that is has low precision and that it requires a high level of experience for proper evaluation of the color change of the seed when using visual (eye) inspection. Therefore this method cannot be easily performed by simple visual inspection.

In addition there is the method of inspecting electrical conductivity which involves the measurement of ions (charged substances) discharged from seed to check seed viability. However, it is not possible to detect seed viability on the basis of individual seeds. Rather, the method requires that a bulk of seeds is checked by determining the electrical conductivity of electrically charge substances discharged from the seed. Thus, this method can only be used to assess bulk seed quality and to compare the quality of batches of seeds.

There is also the method of using fluorescent substances such as sinapine or amino acids discharged from cruciferous vegetable seeds, wherein seeds are allowed to absorb water and are coated with cellulose before drying, and wherein the fluorescent substances such as sinapine or amino acids on the cellulose coat are detected, either by fluorescence measurements or by staining using the ninhydrin reaction which allows for visual inspection. Although such a method is essentially non-destructive, and allows the assessment of viability of individual seeds, it has the shortcoming that it is a complicated process and that it is difficult to automate this process so that it can be performed by machinery.

### SUMMARY OF THE INVENTION

Therefore the purpose of this invention is to provide a method for the detection of seed viability using a short and simple procedure.

The present inventors have found that when dipping seed into a suspension comprising resazurin and a fermenting or respiring microorganism such as yeast, the viability of the seed in said suspension is revealed by a color change of the resazurin in said suspension. Without wishing to be bound by theory, it is believed that this is the result of the respiration or fermentation of the metabolites such as carbohydrate, amino acids and so on, that have leached out of the seed, and which are metabolized by the fermenting or respiring microorganism, and which metabolization results in a change in the redox potential of the suspension, changing the color of the resazurin. Of particular interest to the present invention is the use of resazurin as a redox indicator for detecting microbial growth in aqueous suspensions containing seed and microorganisms. An easily perceptible color change from blue (oxidized - resazurin) to red (reduced - resorufin) indicates bacterial growth and, therefore, the presence of growth substrates leached out from the seed, which is indicative of a seed having reduced viability.

The present invention is useful both for agriculture or for seed industry such as distribution and storage since it allows the determination of seed viability, either by observation by eye or by measurement using a device capable of detecting color changes in aqueous media. The method is nondestructive, rapid and simple, and allows for the inspection of seed quality.

In a first aspect, the present invention provides a method for analyzing seed viability comprising incubating at least one seed in an aqueous solution comprising resazurin and a respiring or fermenting microorganism, and monitoring in said aqueous solution the reduction of the resazurin to resorufin as a result of growth of said microorganism, wherein said seed exhibits reduced viability in case that said reduction is observed.

In a preferred embodiment of a method of the invention, the respiring or fermenting microorganism is yeast.

In another preferred embodiment of a method of the invention, the resazurin is present in said aqueous solution in an amount of 50-150 µg/mL.

In yet another preferred embodiment of a method of the invention, the yeast is present in said aqueous solution in an amount of 0.1-10 mg/mL, preferably 1-4 mg/mL.

In still another preferred embodiment of a method of the invention, the aqueous solution is distilled water.

In another preferred embodiment of a method of the invention, the step of monitoring in said aqueous solution the reduction of the resazurin to resorufin as a result of growth of said microorganism involves detecting whether the color of said aqueous solution changes from blue to red or not.

In another preferred embodiment of a method of the invention, the aqueous solution is provided in the wells of a microtiter plate, and wherein individual seeds are placed in said wells.

In still another preferred embodiment of a method of the invention, the reduction of the resazurin to resorufin is detecting by determining the optical density of said aqueous solution at 570 nm using an optical density or absorption reader, or by determining the fluorescence using a fluorometer capable of producing light excitation of 540-560 nanometers and detecting fluorescence emission of 580-600 nanometers, or by quantifying the color change form blue to red of resazurin using an imaging device.

Disclosed is a composition for detecting seed viability comprising an aqueous solution comprising resazurin and yeast, wherein said resazurin is present in an amount of 50-150 µg/mL and wherein said yeast is present in an amount of 1-4 mg/mL. Inhibition of autoreduction of resazurin may be achieved by the addition of methylene blue as described in US Patent 5,501,959. The invention thus relate to a resazurin reagent composition as defined herein as agent for detecting seed viability. The said solution is used by dipping seed into such a solution, then checking the change in color according to the seed viability, and finally observing this by visual (eye) inspection or by using specific machinery in the form of readers. More desirably, the composition for detecting seed viability may be used by mixing resazurin of 50 µg/mL density and yeast of 4 mg/mL density.

shown is also a computer program product either on its own or on a carrier, which program product, when loaded and executed in a computer, a programmed computer network or other programmable apparatus:
- puts into force a method of determining the optical density or absorbance of an aqueous solution at 570 nm using an optical density or absorbance reader, or a method of determining the fluorescence of an aqueous solution using a fluorometer capable of producing light excitation of 540-560 nanometers and detecting fluorescence emission of 580-600 nanometers, or a method of quantifying the color change form blue to red of resazurin using an imaging device, wherein said optical density, absorbance, fluorescence or color change of said aqueous solution is stored in a memory module of said computer, programmed computer network or other programmable apparatus for comparison to standard reference values for said optical density, absorbance, fluorescence or color change, or
- receives input data on the optical density, absorbance, fluorescence or color change of an aqueous solution comprising resazurin for comparison to standard reference values for said optical density, absorbance, fluorescence or color change in said solution;
- which program product compares the optical density, absorbance, fluorescence or color change of said aqueous solution with said standard reference values;
- wherein said standard reference values are stored in a memory module of said computer, programmed computer network or other programmable apparatus,
- and wherein said standard reference values are obtained by performing a method according to claim 8 for at least two time points during said incubation.

The method of the invention can very suitably be performed using a microtiter plat, such as a 96 well plate (e.g. 96 well clear polyethylene microplate), and placing individual seed inside a well immersed into the resazurin reagent composition as defined herein, then detecting or scanning the level of absorption by visual observation or by using a multiplate reader in order to detect whether the resazurin changes from blue to red color or not, and finally detecting and analyzing color density of scan image.

It is to be understood that the term respiring or fermenting microorganism in a resazurin reagent composition as defined herein may include any type of cell that respires, ferments, grows or shows metabolic activity in an aqueous solution of resazurin at the concentrations indicated herein, that results in the reduction of resazurin. It is to be understood that a plurality of cells is generally meant by the term in the context of the present invention. Such a plurality may be referred to as a culture. The respiring or fermenting microorganism may include a mammalian cell, a microbial cell, including protozoans, algae, fungi, bacteria, and yeast. More preferably the respiring or fermenting microorganism is a yeast, most preferably *Saccharomyces cerevisiae.*

It is desirable to stimulate the color change of the resazurin by placing the container wherein the seed is incubated in the resazurin reagent composition (such as the 96 well plate) into an incubator at a temperature of about 20-40°C. Most desirably the temperature is maintaining at 35°C, which temperature is optimal for activation of yeast. The incubation time before a color change is observed is suitably 2-4 hours, depending on the size of the seed and the quantity of metabolites discharged. Generally 4 hours for cabbage and 2 hours for radish are sufficient. It will be understood that shorter periods such as 5, 10, 30, or 60 minutes of incubation combined with very sensitive methods for detecting resazurin reduction may also be used. Likewise, longer incubation periods, such as overnight incubations are also possible.

The analysis of the color change can be analyzes more precisely by using mechanical devices for detecting of color intensity. Such devices may include image scanning devices combined with image analysis equipment and software for digital image analysis or by detecting light transmission, light extinction or fluorescence by dedicated optical illumination and reading devices such as multiplate readers. Above devices may include a computer program product according to the invention which either performs the analysis of the presence of color change or infers from data provided to said program whether the analysis is indicative of a viable or non-viable seed.

### BRIEF DESCRIPTION OF THE DRAWINGS

Figure 1 describes in a diagram the method of the present invention for detecting and analyzing seed viability by using the composition of the present invention which composition comprises resazurin and a fermenting or respiring microorganism as defined herein.

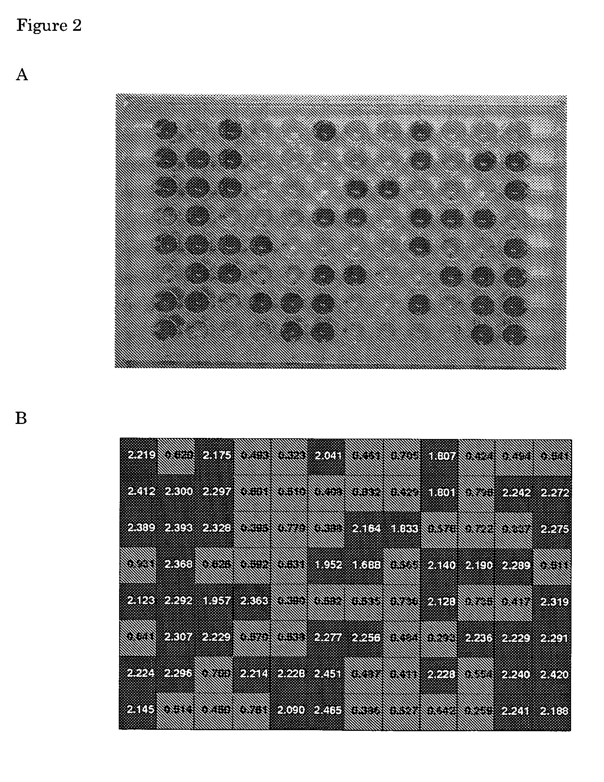

Figure 2 shows the result of the test as described in the Example below, wherein the resazurin color in a 96-well plate is shown in response to the presence of viable (normal) cabbage seeds and nonviable (aged) cabbage seeds, following 4 hours of incubation at 35°C in the presence of the resazurin reagent composition as defined herein. Panel A: Photograph of 96 well plate with 48 viable and 48 non-viable seeds after incubation showing the decoloration of resazurin in the wells of particular seeds. Panel B: Schematic indication of the absorption values measured in the different wells of the plate of panel A.
Figure 3 shows the time course OD change of the resazurin reagent composition at 570 nm in response to the presence of normal (viable) and aged (nonviable) cabbage seeds.

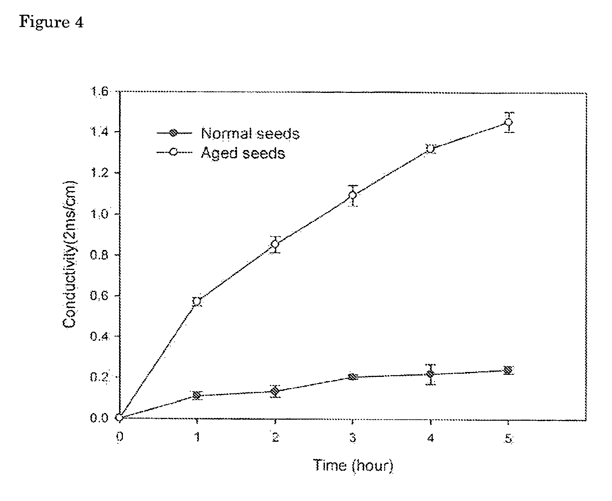

Figure 4 shows the conductivity changes of normal and aged cabbage seeds.
Figure 5 shows a scanned image of an experiment using the method of the invention showing the color change pattern in the resazurin reagent composition as described herein in response to seed viability.

### DETAILED DESCRIPTION OF THE INVENTION

The inventive concept of the present invention is to reduce resazurin in a cell suspension of cells, through the respiration, fermentation, metabolic activity or growth of said cells, preferably yeast cells, which respiration, fermentation, metabolic activity or growth is a direct result of the presence of metabolic substrates for respiration, fermentation, metabolic activity or growth of said cells in the cell suspension, which substrates leak from a seed in the case that said seed is nonviable, and wherein viable seeds will not result in resazurin reduction because they do not provide said metabolic substrates to the cells.

Resazurin (blue color) is an oxazon which readily gives off its oxygen and is reduced to an oxazin-resorufin (red color). Resazurin is generally applied as an oxidation-reduction (redox) indicator in biochemistry. Moreover resazurin is also used as pH indicator, exhibiting a blue color at pH values above 6.8 and exhibiting a red color at pH values below 5.3. It has also been used for biochemical testing of the microbiological quality of milk. This particular test is based on the reduction of resazurin by the respiratory activity of bacteria contained in milk, whereby the quality of the milk is determined according to the degree of decolorization (blue to red) of the milk after addition of resazurin.

During the progression of seed degeneration, the cell membrane of the seed is destroyed, and when such a seed absorbs water, carbohydrates, amino acids, proteins and inorganic substances will not be retained by the cell membrane, but are discharged to the exterior. The degree of cell membrane destruction is proportional to the level of seed degeneration, and hence also proportional to the amount of metabolites discharged from said seed. Viable seeds, in contrast, have an intact cell membrane, and when such seed are dipped into water, there is no metabolite discharge. It has now been found that the discharge of metabolites from seed stimulates the growth of yeast, which in turn reduces resazurin and results in a color change that allows the determination of the state of the seed viability.

Therefore the purpose of this invention is to provide a method for detecting by visual inspection or by use of specific readers precisely whether the seed is viable through a simple and short procedure by which a solution of resazurin and yeast are added to the seed.

When microorganisms and mammalian cells grow in a growth medium, they convert nutrients to energy, resulting in a chemical reduction of their environments. An oxidation/reduction indicator which is present in the environment of the growing microorganism or cells will also be reduced. Thus, the use of an oxidation/reduction indicator with an appropriate oxidation potential range provides a universally applicable test for growth of all microorganisms and mammalian cells. Resazurin is such an oxidation/reduction indicator and is reduced to resorufin. Resazurin is deep blue in reflected color and nonfluorescent. Resorufin is red and highly fluorescent. This reduction of resazurin to resorufin is the basis for the method of detecting seed viability by visible light reading or fluorescence excitation reading in a method of this invention.

The present invention relates to a method to detect seed viability using a resazurin reagent composition comprising resazurin and a respiring or fermenting microorganism. By immersing seed into a resazurin reagent composition as defined herein, and observing the color of resazurin in solution changing from blue to red, the seed viability can be determined by visual inspection as well as by analyzing images of the said solution surrounding the seed via computer upon scanning the container or containing the seed for a color change or by detecting the level of resazurin extinction (OD).

Moreover seed viability can be detected by a simple and short procedure without destroying the seed. Therefore the present allows for the analysis of seed viability in advance, that is before sowing of that seed, which is very useful and applicable to either agricultural or seed industry such as distribution and storage.

In general, any microorganism or cell using carbohydrate or another seed leachate metabolite, such as amino acids, peptides, proteins, fatty acids, etc. as a nutrition source can be used in a method of the invention. Yeast is preferred, as is can be easily stored when dry and reactivated upon suspension in water. With seed leachate is meant the complete set of primary and secondary metabolites that are discharged from the seed by what is known as seed leakage, including the primary metabolites such carbohydrates (composed of sugars), lipids (most contain fatty acids), proteins (composed of amino acids), and nucleic acids (DNA and RNA, made up of nucleotides) and the secondary metabolites such as alkaloids, terpenoids, and phenolics.

The resazurin reagent composition for detecting seed viability can be prepared using commercially available resazurin (e.g. Sigma company). The yeast as used in the examples below was *Saccharomyces cerevisiae* (YSC2, Bakers yeast, Type II) purchased from Sigma company. Good results may also be obtained using other types of yeast.

It is convenient to prepare a resazurin reagent composition comprising resazurin and a respiring or fermenting microorganism as a standard solution for detecting seed viability by mixing resazurin and yeast into distilled water.

Very suitable concentrations of resazurin are between about 50-150 µg/mL. When concentration of resazurin is higher than optimum concentration, the reaction speed is slow. If concentration is too low, the color change is more difficulty to detect. Very good results have been obtained at 50 µg/mL of resazurin.

Very suitable concentrations of yeast are between about 0.1 and 10 mg/mL, preferably between 1-4 mg/mL. When the concentration of yeast is higher than optimum concentration, the reaction speed is too rapid to detect. If the concentration is too low, the color change is also too slow to detect. Very good results have been obtained with yeast at 4 mg/mL.

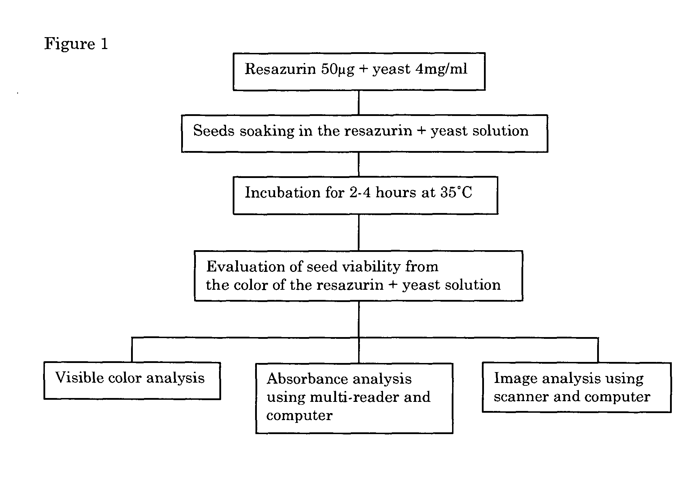

The detection of seed viability using a standard resazurin reagent composition as described above can be performed as follows (see Figure 1).

A resazurin reagent composition as described herein is placed in a container, such as a well of a 96 well plate. A volume of about 150-500 µL of solution for each well is generally sufficient. A seed is also placed in the container and the container is incubated at 20-40°C, preferably about 35°C for yeast. During this incubation, the color of the resazurin is checked at least once or at regular intervals. When the color has changed from blue to red in a control experiment (comprising a control using dead seeds optionally in combination with a control using viable seeds) the time required for that color change is marked and can be used for later experiments as a standard incubation time.

As indicated, for observation of color change, the temperature of incubation is suitably between a range of 20-40°C. If the temperature is too low, the reaction becomes too slow. If the temperature is too high, the reaction is too rapid which is undesirable. The time for color change is different for each seed, for cabbage seed, it is possible to clearly detect the result with the naked eye after 4 hours. The resazurin reagent composition remains blue for normal, viable seed, while it changes into a red color for degenerate seed. Hence degenerate seed can easily be detected.

For automated analysis, a reader may read the optical density (OD) or absorbance at 570nm. A suitable multiplate reader for reading 96 well plates is for instance the Opsys MR (DYNEX Technology USA). Such readers can detect the optical density or absorbance of resazurin for individual seeds in individual wells. The result of the above optical density of each well in a 96 well plate can be read at one time by using related software (Revelation Quick Link Software) in a computer, and can be categorized to provide different levels of seed quality. For instance, as indicated in the Example below, the level of color change in a standard incubation period can reveal whether the seeds are germination seed, non-germination seed and abnormal germination seed. Such distinction can be based on pilot experiments of resazurin reduction by seeds of different quality (levels of viability), and the checking the germination capacity of said seed by performing growth experiments. It is an important advantage of the present invention that these two techniques can be performed on a single seed, because the method is non-destructive. Thus, after inspection of the growth results, the germination characteristics of each seed are compared with the degree of color change in the resazurin reagent composition to provide a correlation there between. Such correlations can be described by a simple standard curve or correlation coefficient by which a computer program can then compute the germination characteristic, in a process wherein the provision of the color change produced by the seed is used as an input for the computer program, and whereby the computer program provides as an output the germination characteristic corresponding to that color change by comparison with the standard curve or by calculation using the correlation coefficient.

As shown in table 2 in the Example below, there is difference in the optical density depending on the type of seed. The optical density after a standard incubation period decreases in the order of normal seed, abnormal seed and non-germination seed. The substance discharged from seed is discharged much in the same order. In all, the result shows that the seed quality bears strong relation with the quantity of discharged substance.

The skilled person will understand that a computer program can be used for analysis wherein as an input the value of the optical density after a standard incubation period is provided, wherein the computer program compares this input with a standard table wherein the optical density after a standard incubation period is annotated to a seed quality characteristic such as viability or germination capacity such as that of table 2 in the Example below, and wherein the computer program provides as an output the corresponding seed quality characteristic.

The seed quality characteristic can suitably be selected from the group consisting of viability, vitality, germination rate, germination energy, germination capacity, stress resistance and vigour.

The term "germination capacity" refer to the percentage of seeds that after a defined period (e.g. 7 days) under optimum conditions give a full seedling. For determining the germination capacity a representative sample is needed. Sampling and the method of determining the germination capacity are described in the handbook for sampling of the International Seed Testing Association (ISTA). The optimum temperature for germination of tomato seed is 23° C. A high germination capacity of the seed does not by definition mean a high field emergence. A better measure for that is the vigour. Investigating germination capacity under unfavourable ambient conditions (e.g. an increased or reduced temperature) can provide information on the vigour of the seed.

The "germination energy" is a measure for the rate of germination and the vitality of the seed. The germination energy is normally determined in the same manner as the germination capacity, but in a shorter period (e.g. 3 days). Seed has a high germination energy if a radicle emerges from a high percentage of the seeds. Germination energy accordingly concerns solely the capacity of the seed to germinate, whereas germination capacity refers to the capacity of the seed to grow well, after germination, into a small seedling. Research into the germination energy under unfavourable or suboptimal ambient conditions (e.g. an increased or reduced temperature) can provide information on the stress resistance of the seed.

The term "stress resistance" or "stress tolerance" refers to the capacity of seed to come to germination under suboptimal conditions or after shorter or longer periods of unfavorable conditions. Measurement of the stress resistance of seed can be done by determining the germination energy under suboptimal conditions.

The term "germination rate" refers to the period (in days or hours) within which up to 50% of seeds come to germination.

The term 'vigour' is normally used to refer both to the capacity of the seed to germinate under suboptimal (stress) conditions and to the capacity of the seed to germinate and to grow out to form an autotrophic seedling under suboptimal conditions. As such, the term hence encompasses the germination energy as well as germination capacity under suboptimal conditions. It is pointed out with emphasis that the term "vigour" as used herein refers to the capacity of seed under suboptimal conditions still to exhibit a good germination and to grow out into an autotrophic seedling and crop, i.e. germination capacity under suboptimal conditions. As used herein, the term relates to germination capacity in the same way as stress resistance relates to germination energy.

In principle, any plant seed may be used in a method according to the invention. Very suitable are crop and vegetable seeds, including seeds of wheat, oats, barley, maize, rye, millet, rice, soy, rapeseed, linseed (flax), coleseed, sunflower, carrot, salsify, scarlet runner, dwarf bean, runner bean, green bean, French bean, broad bean, (garden) pea, lupine, tomato, paprika, pepper, (water) melon, pumpkin, cucumber, aubergine, courgette, onion, leek, lettuce, endive, spinach, lamb's lettuce, gherkin, white cabbage, red cabbage, savoy cabbage, conical cabbage, Chinese cabbage, pakchoi, headed cabbage, cauliflower, Brussels sprouts, sugar beet, beetroot, kohlrabi, chicory, succory, artichoke, asparagus, broccoli, celeriac, blanched celery, radish, grass and herbs.

Hereinafter the method of the present invention is exemplified by means of various practical examples. The scope of the invention is however in no way limited thereto.

### EXAMPLE

This Example shows a simple, nondestructive, and direct detection method for classifying viable and nonviable seeds by visible color and also absorbance measurement according to the color developing with the resazurin reagent composition. The method is single seed based.

### Materials and methods

In this experiment, normal viable seeds of Chinese cabbage (*Brassica campestris* L.), variety Hwangbok (Nongwoo Bio company, Korea) were used to established a methodology for distinguishing between dead and live seeds. The seeds were divided into two lots. One lot was kept as control (normal seeds), that had 100% germination, and another was artificially aged (aged seeds) for 0% germination.

*Artificially ageing treatment*: A predetermined, calculated amount of water was added to seeds with 100% germination rate in order to adjust their moisture content to 20%, then vacuum sealed in a plastic bag and the sealed bag was submerged in a 45 °C water bath for 72 hours. Seeds were brought to their original weight by drying them in a 20°C incubator. These artificially aged seeds were stored at 4 °C for future use.

*Preparation of resazurin reagent composition*: Resazurin (Sigma) was prepared as a stock solution of 1 mg of resazurin/ml. Yeast (*Saccharomyces cerevisiae*) (Sigma, YSC2, baker's yeast, type2) was diluted in distilled water to provide a suspension of 1 mg yeast/ml and was freshly prepared prior to use. A mixture of the resazurin stock solution and yeast suspension was prepared to provide a "resazurin regent" having a final concentration of 50 µg/ml of resazurin and 400 µg/ml of yeast. This resazurin reagent composition was optimized as described herein below for a 96-well microtiter plate having an amount of 150 to 170 µl of the composition in each well depending on seed size.

*Seed testing method*: To evaluate an unknown seed sample, 48 normal cabbage seeds and 48 artificially aged seeds were mixed and individual seeds were placed into each well of a 96-well plate containing 150 µl of the resazurin reagent composition. Three replicate plates were prepared. The plates were put into a 35 °C incubator until the color of the agent was developed depending on the seed viability. After the required incubation time, the wells that remained blue in color were considered to contain normal seeds, while those that turned pink or colorless were considered to contain dead seeds. For further detailed seed quality analysis, the optical density (OD) of the suspension in the wells was measured at 570 nm with a microtiter plate reader (Opsys MR, Dynex Technology, USA) and the seeds were taken out from the wells and sown (planted) on blotters at a position corresponding to their position in the 96 well plate, and the sown seed were evaluated as either representing normal (normal germination), abnormal (abnormal young plant) or dead seeds (non-germinating).

In a separate experiment, in addition to the Chinese cabbage seeds described above, three other cabbage (*Brassica oleracea* L.) varieties, cv. Dynamic, Tara, and Hongwaljuk, and two radish (*Raphanus sativus L*.) varieties, cv. Jangbaek and R-347 (Nongwoobio seed Co.) were used to evaluate intact seeds of unknown quality. The seeds were soaked in 150 µl of the resazurin reagent composition in a 96-well plate and incubated in 35°C for 2-4 hours and the OD at 570 nm was recorded as described above.

### Results and discussion

Preliminary experiments were carried out on seeds of the Chinese cabbage 'Hwangbok', using normal and artificially aged seeds. Initially, the seeds were examined after immersion for various times of 1, 2, 3, 4, 5 and 6 hours in the resazurin reagent composition. The reagent composition was prepared by dissolving the resazurin at 50 µg/ml in distilled water and yeast was added to the solution to provide for various final concentrations of 0.1, 0.2, 0.3, 0.4, and 0.5 mg/ml of yeast in the final reagent composition. Individual seeds were immersed in 150 µl of resazurin reagent composition contained in each well of a 96-well plates and the plates were placed into different incubators set at 20, 25, 30 and 35 °C. It was found that the original color of the composition, which was violet-blue, did not change in the presence of normal seeds. However, the color gradually changed to a faint pink and colorless in the presence of artificially aged seeds during incubation. This indicated that the resazurin was reduced in the presence of the aged seeds. The color change was more rapid, and wells were pink to colorless at an earlier time point, with increasing concentrations of yeast. Also the color change was more rapid at higher temperatures. From these preliminary tests, it was concluded that a yeast concentration of about 400 µg/ml in 50 µg/ml of resazurin and an incubation time of 4 hours in the 35 °C were optimal, since the resazurin reduction was the most rapid and was also clearly visible to the eye under these conditions. Also from the tests of various resazurin concentrations, 50 µg/ml of resazurin was found to be optimal, since at higher concentrations, the reduction was slowed and time to discoloration was prolonged. Subsequently, a sample of 48 normal and 48 aged seeds of Chinese cabbage 'Hwangbok' were mixed thoroughly and the individual seeds were immersed in the resazurin reagent composition in 96-well plate under these optimal test conditions. The results are displayed in Table 1. Table 1 shows normal and aged cabbage seeds (Hwangbaek) and corresponding color groups and OD at 570 nm depending on resazurin reduction.

**Table 1. Normal and aged cabbage seeds (Hwangbaek) and corresponding color groups and OD at 570 nm depending on resazurin reduction. (Average of three 96-well plate)**

| Variety | Color group | OD ± s.d. | Normal Seeds (%) | Aged Seeds (%) |
|---|---|---|---|---|
| | Blue | 2.379±0.02 | 100 | 0 |
| Whangbaek | Pink | | | |
| | Colorless | 0.636±0.21 | 0 | 100 |

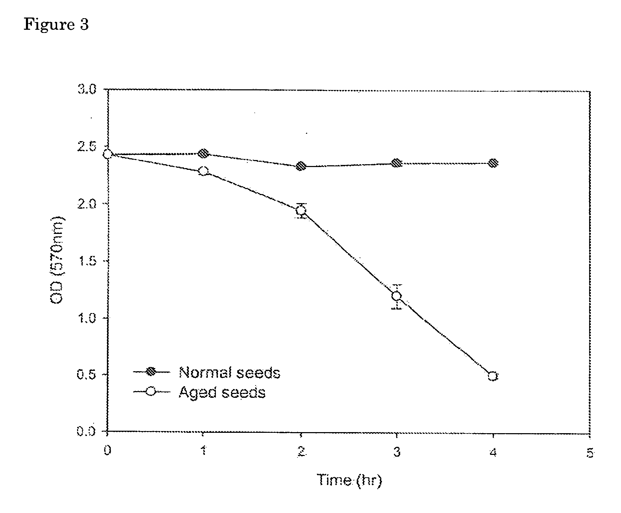

Figure 1 shows the 96-well plate changed in color after 4 hours incubation at 35°C. Time course OD changes were recorded at 570 nm for five hours (Fig. 2). The classification accuracy of normal and aged seed was 100% from the resazurin color (Table 1). One replication of 96-well plate containing 48 normal and 48 aged seeds showed exactly 48 blue (normal seeds) and 48 colorless (aged seeds), the same as three replications (Fig. 1). The OD of the resazurin reagent composition recorded at 570 nm showed clearly decreased from the aged seeds compared to that from normal seeds (Fig 2). Also, the OD had a good relationship between conductivity, as the conductivity of the resazurin from the aged seeds was dramatically increased compared to that of normal seeds (Fig 3). In this respect, it is very clear for the resazurin to be rapidly reduced by the high leakage from the aged seeds. As the compound of the leachate comprises sugar, amino acid and other nutritional materials (Abdul-Baki and Anderson, 1970; Min, 1995; Min, 2000) for yeast, it would be assumed that the yeast used the leachate as a nutritional materials for activating metabolization.

After the optimal testing condition had been determined, the validity of the method was tested on three samples of cabbage seeds of the 'Dynamic', 'Tara' and 'Holwaljuk', and two samples of the radish seeds 'Jangbaek' and 'R-347' having different germination percentages. The color of the resazurin reagent composition changed faster in radish than in cabbage. Therefore, it was found that the incubation comprising soaking in a resazurin reagent composition comprising 50 µg/ml resazurin at 35°C of cabbage seeds for 4 hours and the radish seeds for 3 hours was the optimal incubation time.

The seeds were removed from the resazurin reagent composition and were classified into three groups according to color (blue, pink and colorless) of the reagent composition; each group being germinated separately in blotters. The OD of the resazurin was recorded by a multi-plate reader after removing the seeds from the wells. The germination capacity and corresponding color groups of intact cabbage and radish seeds after treatment with resazurin reagent composition were determined and are shown in Table 2. It was found that intact cabbage and radish seeds respond to the resazurin reagent composition and the color intensity from the resazurin could be used as a reliable indicator of germinability.

**Table 2. The OD₅₇₀ of the resazurin reagent composition upon incubation with various seed qualities of 3 cabbage varieties and 2 radish varieties.**

| **Types of seed** | **Normal seed** | **Abnormal seed** | **Non-germination seed** |
|---|---|---|---|
| Dynamic cabbage | 1.960 ± 0.03 | 1.491 ± 0.06 | 0.657 ± 0.03 |
| Tara cabbage | 2.045 ± 0.02 | 1.571 ± 0.05 | 0.725 ± 0.02 |
| Hongwoljok cabbage | 2.060 ± 0.02 | 1.608 ± 0.04 | 1.036 ± 0.03 |
| Changbaek radish | 1.274 ± 0.03 | 0.997 ± 0.03 | 0.654 ± 0.03 |
| R-347 radish | 1.566 ± 0.02 | 1.302 ± 0.05 | 0.869 ± 0.04 |

As shown in table 2, there is difference in the OD depending on the type of seed. The OD is lower in the order of normal seed, abnormal seed and non-germination seed. The substance discharged from seed is discharged much at the same order. Taken together, the result shows that the seed quality can be accurately measured using the method of the invention.

Based on above result, we used a computer program for analysis of the seed quality using the results in table 2 for individual vegetable varieties as standard reference values for specific test conditions. By determining a test value for a seed, and comparing it to the standard reference values, it was possible to analyze the seed quality swiftly and obtain prompt results.

### Checking according to color difference using resazurin reagent composition

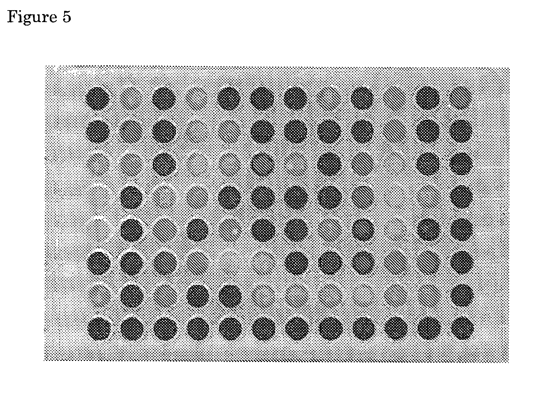

Again, we scanned a color-change 96 well plate and analyzed the image captured by an imaging device. A representative image is shown as in Figure 5. The result obtained with the image is exactly same as the result obtained by detecting the optical density. The analysis of the image need not necessarily be done by scanning the 96 well plate, but it is also possible to use a digital photograph as shown in Figure 2A. However scanning by scanner is more advantageous for its convenience to conduct. Image analysis can be performed by using software (SigmaScan Pro 5.0 Systat Software, Inc. USA) for image analysis of a scanned photograph by opening the image file in a computer program, and calculate directly the number or percent according to the color differentiation or color intensity on such image.

## Claims

1. Method for analyzing seed viability comprising incubating at least one seed in an aqueous solution comprising resazurin and a respiring or fermenting microorganism, and monitoring in said aqueous solution the reduction of the resazurin to resorufin as a result of growth of said microorganism, wherein said seed exhibits reduced viability in case that said reduction is observed.

2. Method according to claim 1, wherein said respiring or fermenting microorganism is yeast.

3. Method according to claim 1 or 2, wherein said resazurin is present in said aqueous solution in an amount of 50-150 µg/mL.

4. Method according to any one of claims 1-3, wherein said yeast is present in said aqueous solution in an amount of 1-4 mg/mL.

5. Method according to any one of the preceding claims, wherein said aqueous solution is distilled water.

6. Method according to any one of the preceding claims, wherein said step of monitoring in said aqueous solution the reduction of the resazurin to resorufin as a result of growth of said microorganism involves detecting whether the color of said aqueous solution changes from blue to red or not.

7. Method according to any one of the preceding claims, wherein said aqueous solution is provided in the wells of a microtiter plate, and wherein individual seeds are placed in said wells.

8. Method according to any one of the preceding claims, wherein the reduction of the resazurin to resorufin is detecting by determining the optical density of said aqueous solution at 570 nm using an optical density reader, or by determining the fluorescence using a fluorometer capable of producing light excitation of 540-560 nanometers and detecting fluorescence emission of 580-600 nanometers, or by quantifying the color change form blue to red of resazurin using an imaging device.

## Patentansprüche

1. Verfahren zur Analyse der Lebensfähigkeit von Saatgut, umfassend Inkubieren von zumindest einem Saatgut in wässriger Lösung, umfassend Resazurin und einen atmenden oder gärenden Mikroorganismusund Beobachtung der Reduktion des Resazurin zu Resorufin in der wässrigen Lösung als ein Ergebnis des Wachstums des Mikroorganismus, wobei das Saatgut reduzierte Lebensfähigkeit aufweist, wenn die Reduktion beobachtet wird.

2. Verfahren nach Anspruch 1, wobei der atmende oder gärende Mikroorganismus Hefe ist.

3. Verfahren nach Anspruch 1 oder 2, worin das Resazurin in der wässrigen Lösung in einer Menge von 50 - 150 µg/ml vorhanden ist.

4. Verfahren nach einem der Ansprüche 1 bis 3, wobei die Hefe in der wässrigen Lösung in einer Menge von 1 - 4 mg/ml vorhanden ist.

5. Verfahren nach einem der vorangehenden Ansprüche, wobei die wässrige Lösung destilliertes Wasser ist.

6. Verfahren nach einem der vorangehenden Ansprüche, wobei der Schritt des Beobachtens der Reduktion von Resazurin zu Resorufin in der wässrigen Lösung als das Ergebnis des Wachstums des Mikroorganismus, das Detektieren einschliesst, ob die Farbe der wässrige Lösung von blau zu rot wechselt, oder nicht.

7. Verfahren nach einem der vorangehenden Ansprüche, wobei die wässrige Lösung in den Vertiefungen einer Mikrotiterplatte bereitgestellt wird, und wobei ein einzelnes Saatgut in eine Vertiefung gelegt wird.

8. Verfahren nach einem der vorangehenden Ansprüche, wobei die Reduktion von Resazurin zu Resorufin durch Bestimmen der optischen Dichte der wässrigenLösung bei 570 nm unter Verwendung eines optischen Dichtemessers detektiert wird, oder durch Bestimmen der Fluoreszenz unter Verwendung eines Fluorometers, das fähig ist, eine Lichtanregung bei 540 - 560 Nanometern zu erreichen und Detektieren einer Fluoreszenzemission von 580 - 600 Nanometern, oder durch Quantifizieren der Farbänderung von blau zu rot des Resazurins unter Verwendung einer bildgebenden Vorrichtung.

## Revendications

1. Procédé pour analyser la viabilité de graines comprenant l'incubation d'au moins une graine dans une solution aqueuse comprenant de la résazurine et un micro-organisme respirateur ou fermenteur, et la surveillance dans ladite solution aqueuse de la réduction de la résazurine en résorufine en conséquence de la croissance dudit micro-organisme, ladite graine présentant une viabilité réduite dans le cas où ladite réduction est observée.

2. Procédé selon la revendication 1, dans lequel ledit micro-organisme respirateur ou fermenteur est la levure.

3. Procédé selon la revendication 1 ou 2, dans lequel ladite résazurine est présente dans ladite solution aqueuse en une quantité de 50 à 150 µg/ml.

4. Procédé selon l'une quelconque des revendications 1 à 3, dans lequel ladite levure est présente dans ladite solution aqueuse en une quantité de 1 à 4 mg/ml.

5. Procédé selon l'une quelconque des revendications précédentes, dans lequel ladite solution aqueuse est de l'eau distillée.

6. Procédé selon l'une quelconque des revendications précédentes, dans lequel ladite étape de surveillance dans ladite solution aqueuse de la réduction de la résazurine en résorufine en conséquence de la croissance dudit micro-organisme met en oeuvre la détection du fait que la couleur de ladite solution aqueuse vire du bleu au rouge ou non.

7. Procédé selon l'une quelconque des revendications précédentes, dans lequel ladite solution aqueuse est disposée dans les puits d'une plaque de microtitrage, et dans lequel les graines individuelles sont placées dans lesdits puits.

8. Procédé selon l'une quelconque des revendications précédentes, dans lequel la réduction de la résazurine en résorufine est détectée en déterminant la densité optique de ladite solution aqueuse à 570 nm en utilisant un lecteur de densité optique, ou en déterminant la fluorescence en utilisant un fluorimètre capable de produire une lumière d'excitation de 540 à 560 nanomètres et en détectant l'émission de fluorescence de 580 à 600 nanomètres, ou en quantifiant le changement de couleur du bleu au rouge de la résazurine en utilisant un dispositif d'imagerie.
